(19) Europäisches Patentamt / European Patent Office / Office européen des brevets

(11) **EP 4 687 721 B1**

(12) **EUROPEAN PATENT SPECIFICATION**

(45) Date of publication and mention
of the grant of the patent:
**13.05.2026 Bulletin 2026/20**

(21) Application number: **24713975.1**

(22) Date of filing: **29.03.2024**

(51) International Patent Classification (IPC):
*A61B 18/00* (2006.01)   *A61B 18/02* (2006.01)
*A61B 18/14* (2006.01)   *A61B 18/18* (2006.01)
*A61B 18/20* (2006.01)   *A61B 90/00* (2016.01)
*A61B 34/10* (2016.01)

(52) Cooperative Patent Classification (CPC):
**A61B 18/00; A61B 34/10; A61B 34/25;**
A61B 18/02; A61B 18/14; A61B 18/1815;
A61B 18/20; A61B 2018/00577; A61B 2018/00702;
A61B 2018/00791; A61B 2034/101; A61B 2034/102;
A61B 2034/104; A61B 2034/105; A61B 2034/107;
(Cont.)

(86) International application number:
**PCT/EP2024/058738**

(87) International publication number:
**WO 2024/200801 (03.10.2024 Gazette 2024/40)**

(54) **VISUALIZING SEQUENTIALLY AND SIMULTANEOUSLY ACTIVATED APPLICATORS OF AN ABLATION PLAN**

VISUALISIERUNG VON SEQUENTIELL UND GLEICHZEITIG AKTIVIERTEN APPLIKATOREN EINES ABLATIONSPLANS

VISUALISATION D'APPLICATEURS ACTIVÉS SUCCESSIVEMENT ET SIMULTANÉMENT D'UN PLAN D'ABLATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **31.03.2023 EP 23166097**

(43) Date of publication of application:
**11.02.2026 Bulletin 2026/07**

(73) Proprietor: **Koninklijke Philips N.V.**
**5656 AG Eindhoven (NL)**

(72) Inventors:
- **ISOLA, Alfonso Agatino**
  **5656 AG Eindhoven (NL)**
- **HAUTVAST, Guillaume Leopold Theodorus Frederik**
  **5656 AG Eindhoven (NL)**

(74) Representative: **Philips Intellectual Property & Standards**
**High Tech Campus 52**
**5656 AG Eindhoven (NL)**

(56) References cited:
US-A1- 2008 033 420    US-A1- 2014 058 387
US-A1- 2020 022 649    US-A1- 2021 007 805

(52) Cooperative Patent Classification (CPC): (Cont.)
A61B 2034/2065; A61B 2090/365; A61B 2090/374;
A61B 2090/3762; A61B 2090/378

**Description**

FIELD OF THE INVENTION

The present invention is generally related to therapy treatment planning and guidance, and more particularly, to visually representing implementation of an ablation plan.

BACKGROUND OF THE INVENTION

In the field of interventional oncology, percutaneous ablation, including thermal and non-thermal based ablation, is an interventional cancer treatment option that has seen a significant increase in adoption in the past decade, and is predicted to continue to grow, at least in the near term, at a compound annual growth rate of approximately 8-10%. Thermal ablation can be delivered using various ablation modalities, including e.g., radiofrequency ablation (RFA), microwave ablation (MWA), high-intensity focused ultrasound (HIFU), focal laser ablation (FLA), and cryo-ablation. Non-thermal ablation techniques include irreversible electroporation (IRE), which uses non-thermal energy to create permanent nanopores in a cell membrane that disrupts cellular homeostasis and ultimately causes cell damage within an applied electric field.

In clinical practice, these ablation procedures consist of placing one or more ablation applicators inside or near a target region with the help of image guidance. Typically, physicians place these needle-like applicators while inspecting real-time ultrasound or interventional radiology images (CT/MR/CBCT), and determine the intended location for the resulting ablation based on information provided from the manufacturer, results in clinical trials, and personal experience.

Current ablation planning and guidance relies on pre-defined ablation zone dimensions that were determined in ex-vivo or in-vitro experiments in which a single applicator was activated. However, when activated simultaneously, the cumulative effect of nearby applicators causes achieved ablation zones to merge and cover a larger area. For cryo-ablation in particular, this merging is referred to as the "kissing ice-ball" effect that can be visualized with real-time imaging of the tissue under treatment. An often pursued solution for addressing the merging of ablation zones is the use of biophysical modelling techniques relying on Pennes bioheat equation. These techniques have proven to model the achieved ablation zones more accurately, by incorporating detailed information on the applicator devices themselves, and the tissue within which they are inserted. Yet, to be accurate, these techniques tend to require information that is either clinically unavailable or very specific to certain ablation devices. Examples of information that is clinically unavailable (or difficult to obtain) includes patient core temperature, accurate tissue and vasculature segmentation, patient and tissue specific rates of perfusion and heat absorption, convective flow rates in surrounding vasculature, etc. Device specific information includes detail of the physical design of the ablation device including the location of actuators, shielding etc. Consequently, transitioning the use of biophysical models from the investigational realm to the commercial environment is challenging. Additionally, providing more user control in the implementation of an ablation plan that is based on single and composite ablations is needed.

US 2021/007805 teaches a system not allowing for a visual distinction between sequentially and simultaneously activated applicators.

SUMMARY OF THE INVENTION

One object of the present invention is to improve upon existing systems in the implementation and control of ablation therapy plans that use single and composite ablations. To better address such concerns, in a first aspect of the invention, a system is disclosed that receives an ablation plan comprising plural ablation zones corresponding to plural applicators, the plural ablation zones comprising one or more single ablation zones and one or more composite ablation zones, and provides on a display device a representation of the ablation plan and a visual distinction between sequentially activated applicators and simultaneously activated applicators, the plural applicators comprising the sequentially activated and simultaneously activated applicators. By providing visual distinctions between sequentially activated and simultaneously activated applicators, a user may have more control over the implementation of the ablation therapy plan.

In one embodiment, the sequentially activated applicators provide single ablations and the simultaneously activated applicators provide composite ablations, wherein the composite ablations are based on blending implicit functions. Using blending for composite ablations avoids the need for information that is either clinically unavailable or very specific to certain ablation devices function. However, certain embodiments may use composite ablation zones that are determined from biophysical modeling techniques while still benefiting from the visualization of single and composite ablations during implementation of an ablation therapy plan.

In one embodiment, the system prohibits user-requested adjustments to the ablation plan that are unfeasible to implement. For instance, there may be circumstances where the ablation therapy procedure does not go as planned, inviting adjustments by a user. The system ensures that such adjustments are still within the capabilities of the system and/or the chosen applicator(s).

In one embodiment, the system provides a graphical user interface that enables a user to control ablations corresponding to the plural ablation zones of the ablation plan, giving the user the capability to control the progress of the ablation therapy plan and activation/deactivation of applicators.

These and other aspects of the invention will be apparent from and elucidated with reference to the embodiment(s) described hereinafter.

BRIEF DESCRIPTION OF THE DRAWINGS

Many aspects of the invention can be better understood with reference to the following drawings, which are diagrammatic. The components in the drawings are not necessarily to scale, emphasis instead being placed upon clearly illustrating the principles of the present invention. Moreover, in the drawings, like reference numerals designate corresponding parts throughout the several views.

FIGS. 1A-1C are example representations of complex ablation zones that are represented by certain embodiments of an ablation therapy system, in accordance with an embodiment of the invention.

FIG. 2 is schematic diagram that illustrates an embodiment of an example ablation therapy system that facilitates planning, guidance, and control of ablation therapy treatment to a tumor mass or lesion, in accordance with an embodiment of the invention.

FIG. 3 is a schematic diagram that illustrates a group of three applicators arranged in a triangle pattern with a pattern barycenter as the composite ablation entry trajectory origin, in accordance with an embodiment of the invention.

FIGS. 4A-4B are schematic diagrams that illustrate an example of a blended implicit function from two implicit functions based on different blending parameter values, in accordance with an embodiment of the invention.

FIGS. 5A-5C are schematic diagrams that show example composite ablation zones for two-applicator linear patterns alone and in combination with additional groups of applicators, in accordance with an embodiment of the invention.

FIG. 6 is a flow diagram that illustrates an embodiment of an example ablation therapy method, in accordance with an embodiment of the invention.

FIGS. 7A-7B are example screen diagrams that illustrate an embodiment of example graphical user interfaces that enable visual distinctions for sequentially and simultaneously activated applicators for an ablation therapy plan and for more control of ablations, in accordance with an embodiment of the invention.

DETAILED DESCRIPTION OF EMBODIMENTS

Disclosed herein are certain embodiments of an ablation therapy system and method that are used to plan and guide thermal or non-thermal ablation procedures, with an aim to improve user interactions during the treatment process through visualizations enabled through a graphical user interface. The ablation therapy system visually represents the ablation therapy plan (or herein, simply referred to also as an ablation plan) and sequentially and simultaneously activated applicators for single and composite ablations, respectively, providing the user with additional control of an ablation procedure based on segmentations of the cancer lesion(s) and other anatomical structure segmentations.

Digressing briefly, and as explained above, existing ablation planning and guidance relies on pre-defined ablation zone dimensions that are determined in ex-vivo or in-vitro experiments in which a single applicator is activated. However, when activated simultaneously, the cumulative effect of nearby applicators causes achieved ablation zones to merge and cover a larger area. One solution for addressing the merging of ablation zones is the use of biophysical modelling techniques relying on Pennes bioheat equation. These techniques have proven to model the achieved ablation zones more accurately, by incorporating detailed information on the applicator devices themselves, and the tissue within which they are inserted. Yet, to be accurate, these techniques tend to need information that is either clinically unavailable or very specific to certain ablation devices. Certain embodiments of an ablation therapy system use the blending of implicit functions to represent the composite ablation zones, which even if less accurate than biophysical modelling techniques, are unencumbered by the need for detailed information. Further, such techniques are more accurate than existing approaches based on ex-vivo or in-vitro experiments. Also, the visualization of an ablation therapy plan, and in particular, the sequentially and simultaneously activated applicators for single and composite ablations, respectively, provides for improved interactions between the ablation therapy system and the user and more control of implementation of the ablation therapy plan.

Note that reference herein to a composite ablation zone refers to the expected total ablation zone shape delivered by a group or groups of all executed applicators, whether modeled using biophysical modeling techniques or through the use of blending of implicit functions. Also, the term probes and applicators are used interchangeably herein.

Having summarized certain features of an ablation therapy system of the present disclosure, reference will now be made in detail to the description of an ablation therapy system as illustrated in the drawings. While an ablation therapy system will be described in connection with these drawings, there is no intent to limit it to the embodiment or embodiments disclosed herein. Further, although the description identifies or describes specifics of one or more embodiments, such specifics are not necessarily part of every embodiment, nor are all of any various stated advantages necessarily associated with a single embodiment. On the contrary, the intent is to cover alternatives and modifications included within the scope of the disclosure as defined by the appended claims. For instance, two or more embodiments may be interchanged or combined in any combination. Further, it should be appreciated in the context of the present disclosure that the claims are not necessarily limited to the particular embodiments set out in the description.

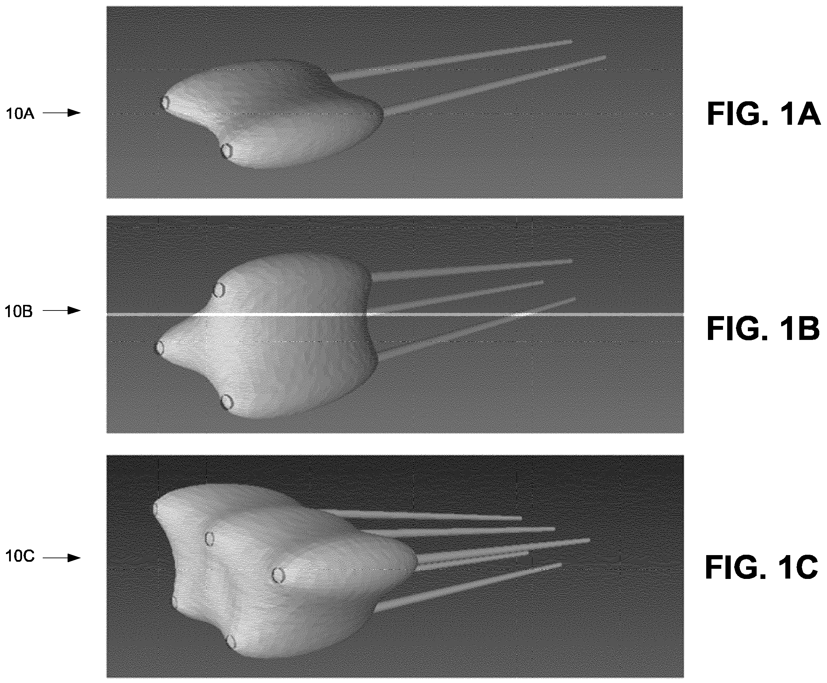

Before commencing further description of features of certain embodiments of an ablation therapy system, a brief discussion of composite ablation zones follows. In the market, different probe models are given with datasheets indicating the expected geometry of both a single ablation zone delivered by a given probe configuration when used in isolation, and also the expected composite ablation zone delivered by different geometrical probe configurations. In some devices, the expected composite ablation zone has an ellipsoidal shape, however there are also other devices, for instance for MWA or IRE treatment, where the composite ablation zone can have quite complex shapes produced via a combination of the multiple ellipsoids delivered by the probes, as depicted by the example complex composite ablation zones 10A-10C in FIGS. 1A-1C, respectively. In contrast to existing systems, certain embodiments of an ablation therapy system as disclosed herein blend implicit functions to represent composite ablation zones during a commissioning phase (or during an implementation phase when there are changes made in the ablation therapy plan), which provides an advance in more accurately representing complex composite ablation zones such as those depicted in FIGS. 1A-1C delivered via multiple groups of probes. However, as indicated above, some embodiments of an ablation therapy system may use composite ablation zones that are modeled using biophysical modeling techniques.

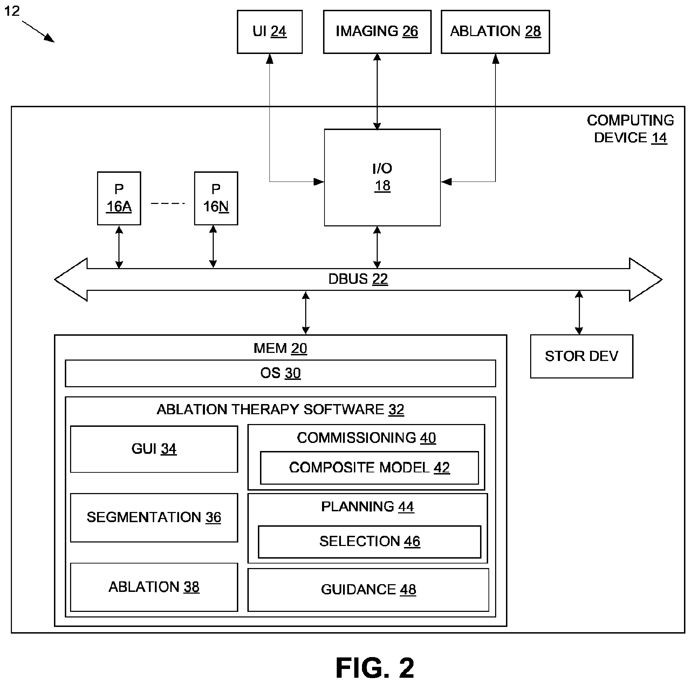

FIG. 2 illustrates an ablation therapy system 12 that facilitates the planning and guidance of one or more ablation protocols to guide treatment of a target region (e.g., a tumor mass or lesion, including any margin) in a subject. Note that reference herein is made to the ablation therapy system 12 responsible for treatment based on the planning and guidance functionality, though in some embodiments, the planning, guidance, and/or treatment may be implemented as separate systems in communication with each other. Successful treatment of large tumors can be achieved by planning ablation probe positions precisely through commissioning with the intent that no part of the tumor is left untreated, and accurately executing the plan. In general, the ablation therapy system 12 provides for planning of ablation therapy, guidance of applicator placement, and therapy ablation treatment based on the planning and guidance. The ablation plan ensures that a majority of (or potentially all) areas of the tumor are covered, and reports the number of ablations involved for an ablation using a particular probe or group(s) of probes. The ablation therapy system 12 also utilizes selection techniques to reduce the number of ablations. Since the plan is quantitative, it can be carried out using a robot and/or by using registered image guidance, such as by quantitatively tracking the ablation probe.

In the depicted embodiment, functionality of the ablation therapy system 12 is implemented as a system of co-located software and hardware components collectively embodied as a computing device 14 (which may include a medical device) and plural sub-systems, wherein the computing device 14 is operatively connected to the plural sub-systems, as described below. In some embodiments, the computing device functionality may be embedded in one of the sub-systems, or one or more of the herein-described sub-system functionality may be integrated into fewer devices. It should be appreciated that, in some embodiments, functionality of the ablation therapy system 12 may be implemented via plural computing devices that are networked at a similar location or situated in different locations, potentially remote from each other, and connected via one or more networks. The computing device 14 includes one or more processors 16 (e.g., 16A...16N), input/output interface(s) 18, and memory 20, etc. coupled to one or more data busses, such as data bus 22. The processor(s) 16 may be embodied as a custom-made or commercially available processor, including a single or multi-core central processing unit (CPU), tensor processing unit (TPU), graphics processing unit (GPU), vector processing unit (VPU), or an auxiliary processor among several processors, a semiconductor based microprocessor (in the form of a microchip), a macroprocessor, one or more application specific integrated circuits (ASICs), field programmable gate arrays (FPGUs), a plurality of suitably configured digital logic gates, and/or other existing electrical configurations comprising discrete elements both individually and in various combinations to coordinate the overall operation of the computing device 14.

The I/O interfaces 18 comprise hardware and/or software to provide one or more interfaces to various sub-systems, including to one or more user interfaces 24, an imaging sub-system 26, and an ablation sub-system 28. The I/O interfaces 18 may also include additional functionality, including a communications interface for network-based communications. For instance, the I/O interfaces 18 may include a cable and/or cellular modem, and/or establish communications with other devices or systems via an Ethernet connection, hybrid/fiber coaxial (HFC), copper cabling (e.g., digital subscriber line (DSL), asymmetric DSL, etc.), using one or more of various communication protocols (e.g., TCP/IP, UDP, etc.). In general, the I/O interfaces 18, in cooperation with a communications module (not shown), comprises suitable hardware to enable communication of information via PSTN (Public Switched Telephone Networks), POTS, Integrated Services Digital Network (ISDN), Ethernet, Fiber, DSL/ADSL, Wi-Fi, cellular (e.g., 3G, 4G, 5G, Global System for Mobile Communications (GSM), General Packet Radio Service (GPRS),etc.), Bluetooth, near field communications (NFC), Zigbee, among others, using TCP/IP, UDP, HTTP, DSL.

The user interface(s) 24 may include a keyboard, scroll-wheel, mouse, microphone, immersive head set, display device(s), etc., which enable input and/or output by or to a user, and/or visualization to a user. For instance, user interface(s) 24, when embodied as a display device, may display a tumor region and plural ablation zones created as part of an ablation therapy plan, with the ablation zones comprising one or any combination of single ablation zones and composite ablation zones. Additionally, the user interface(s) 24 (e.g., display device) may display the positions and orientations of the applicators that are used to form the ablation zones, and visually distinguish sequentially activated (e.g., single ablations) and simultaneously activated (composite ablation zones) during ablation therapy treatment. The user interface(s) 24 may present one or more graphical user interfaces with interface tools to enable a user to visualize and control the activation and display of the applicator activations. In some embodiments, the user interface(s) 24 may cooperate with associated software to enable augmented reality or virtual reality. In effect, the user interface(s) 24, when comprising a display device, enables the display of segmented anatomical structures, including tumors, and the virtual (e.g., simulated) and actual placement of applicators and identification of ablation zones and ablation zone coverage, as well as virtual bounding boxes. In some embodiments, the user interface(s) 24 may be coupled directly to the data bus 22.

The imaging sub-system 26 includes one or more imaging modalities and/or image storage sub-systems that are used to enable visualization of tumors, virtual or actual applicator placement, and/or single or composite ablation zones during pre- and per-operative (e.g., during actual applicator placement and/or ablation treatment) imaging. The imaging sub-system 26 may include ultrasound imaging (e.g., 2D/3D in real-time), fluoroscopy (real-time), magnetic resonance (e.g., MR, in 2D/3D real-time or static), computed tomography (e.g., CT (3D static)), cone beam CT (e.g., CBCT (3D static)), single photon emission CT (SPECT), and/or positron emission tomography (PET). In some embodiments, the images may be retrieved from a picture archiving and communication systems (PACS) or any other suitable imaging component or delivery system.

The ablation sub-system 28 may include any one of various ablation modalities, including radiofrequency ablation (RFA), microwave ablation (MWA), high-intensity focused ultrasound (HIFU), focal laser ablation (FLA), irreversible electroporation (IRE), and cryo-ablation. In some embodiments, software in memory 20 may be used to interface with the ablation sub-system 28 to adjust settings (e.g., probe distance, power, duration), track positioning of the applicators, and/or in the case of robotic-based ablation therapy, control the actual placement and energizing of the applicators.

The memory 20 may include any one or a combination of volatile memory elements (e.g., random-access memory RAM, such as DRAM, and SRAM, etc.) and nonvolatile memory elements (e.g., ROM, Flash, solid state, EPROM, EEPROM, hard drive, tape, CDROM, etc.). The memory 20 may store a native operating system, one or more native applications, emulation systems, or emulated applications for any of a variety of operating systems and/or emulated hardware platforms, emulated operating systems, etc. In some embodiments, a separate storage device (STOR DEV) may be coupled to the data bus 22 or as a network-connected device (or devices) via the I/O interfaces 18 and one or more networks. The storage device may be embodied as persistent memory (e.g., optical, magnetic, and/or semiconductor memory and associated drives). In some embodiments, the storage device or memory 20 may store manufacturer data pertaining to various applicators and applicator patterns.

In the embodiment depicted in FIG. 2, the memory 20 comprises an operating system 30 (OS) (e.g., LINUX, macOS, Windows, etc.), and ablation therapy software 32. In one embodiment, the ablation therapy software 32 comprises a plurality of modules (e.g., executable code) co-hosted on the computing device 14, though in some embodiments, the modules may be distributed across various systems or sub-systems over one or more networks. The ablation therapy software 32 comprises a graphical user interface (GUI) module 34, a segmentation module 36, and an ablation module 38. The ablation therapy software 32 also comprises a commissioning module 40, which includes a composite ablation model(s) or algorithm(s) 42, and a planning module 44, which includes a selection module 46. In one embodiment, the composite ablation model 42, or simply, composite model, comprises implicit functions or algorithms for blending of composite ablation zones. In some embodiments, the composite model 42 may additionally, or alternatively, comprise the known biophysical model or algorithm. The ablation therapy software 32 further comprises a guidance module 48, which through cooperation with the user interface(s) 24, imaging sub-system 26, and ablation sub-system 28, among other modules, guides placement of the applicators via overlay graphics on top of images. Note that functionality of the modules of the ablation therapy software 32 may be shared amongst each other. In some embodiments, there may be fewer or additional modules. For instance, functionality of some modules may be combined, or additional functionality may be implemented yet not shown, such as a communication module, security module, etc.

Functionality of one or more of the various modules is briefly explained here, with further description below. The GUI module 34 provides for reconstruction and rendering on the user interface(s) 24 (e.g., a display device) of image data received from imaging sub-system 26. Objects such as a lesion, organs, critical and/or healthy anatomical structures (or generally, regions not intended for ablation), may be segmented automatically using existing algorithms or by hand with drawing tools along the axes via the segmentation module 36. The segmentation module 36 produces a description of the volumetric regions associated with the specific objects according to existing methods. The GUI module 34, such as through cooperation with the ablation module 38, may also provide visualizations of ablation zones, applicators, and applicator placement for application to one or more target regions, whether virtually placed (e.g., pre-operative) or actually placed (during per-operative implementations). As explained above, and further below, the ablation module 38 and GUI module 34 provide for commissioning GUIs and ablation execution or implementation GUIs that enable a user to control the ablation therapy plan, and to render visually distinctive appearances between simultaneously and sequentially activated applicators.

As explained in part above, the ablation module 38, in conjunction with the GUI module 34, provides for the display of user-selectable options for applicators and/or applicator zones. For instance, the ablation module 38 may store manufacturer data on expected ellipsoidal ablation zones based on different settings (e.g., power, duration, probe separation distance, etc.) for the ablation sub-system 28, and/or pre-defined (e.g., from manufacturer data) applicator patterns (e.g., single probe, two probe, three probe, etc.). In some embodiments, the ablation module 38 may track positioning of the applicators of the ablation sub-system 28 using electromagnetic or other types of sensors on or near the applicators, and/or use image analysis for tracking. The ablation module 38 controls applicators during ablation procedures based on the settings, and in some embodiments, may be used in conjunction with robotic controls to control the placement and activation of the applicators.

The commissioning module 40, in cooperation with the GUI module 34, may enable user-configured placement of applicators and/or ablation zones and/or, optionally, selection of pre-defined placement patterns. The commissioning module 40 further comprises the composite model 42, which comprises functionality (algorithms) used to support non-ellipsoidal composite ablation zones (e.g., such as those shown in FIGS. 1A-1C) based on blending of ellipsoidal zones from the applicators of a group, as explained further below. The commissioning module 40 may operate in a grid or grid-less applicator environment. The commissioning module 40 may provide for fixed positioning (as prescribed by manufacturer data obtained, in one embodiment, by the ablation module 38) or as configured by a user (e.g., clinical user) via the GUI module 34.

The planning module 44 provides for the automated and/or interactive planning of single and composite ablations. For instance, based on segmented anatomical regions, the planning module 44 utilizes the commissioned data to precompute a plurality of (e.g., in some embodiments, all possible) probe configurations from which a preferred solution or plan is selected. The planning module 44 further comprises a selection module 46, which analyzes information associated with the tumor(s) and applicator positioning and ablation zones, and defines a set of ablation positions with orientations. In one embodiment, the selection module 46 comprises existing optimization functionality, which identifies the fewest (or fewer) number of ablations possible that cover the tumor region (e.g., tumor or tumor plus margin). In some embodiments, the selection module 46 identifies the ablation positions with orientations that spare the healthy tissue (e.g., that minimizes or reduces the risk of collateral damage). In some embodiments, additional object volumes are segmented that denote critical regions of tissue or bone that are not to be ablated, and the selection module 46 attempts to generate either the fewest ablations or minimize collateral damage, while also avoiding or reducing the risk of ablation of these regions. In some cases however, the selection module 46 produces unablated areas, whereupon the user is alerted and the regions can be displayed on the user interface(s) 24.

The guidance module 48 provides for user interactive guidance of applicator placement, as described below.

A general description of example operations of the ablation therapy software 32 is described below. After tumor and risk organ segmentation via segmentation module 36, the commissioning module 40 is used to enable commissioning under the direction or instruction of a user (e.g., clinical expert) via GUI module 34. For instance, during commissioning, the clinical expert decides which probes and probe settings to use (e.g., information accessed via the ablation module 38). Probe setting may include probe model, geometry entry pattern (e.g., single probe, two probes in a linear pattern, three probes in a linear or triangular pattern, four probes in a square pattern, etc.), and expected ablation zone (e.g., ellipsoid or complex blended shape). As explained above, expected ellipsoidal ablation zone sizes in manufacturer data sheets are based on either in/ex-vivo experiments on animals or gel phantom mimicking specific tissues of interest. In one embodiment, the ablation therapy software 32 may provide a commissioning GUI (e.g., via GUI module 34) for interaction with a clinical expert for complex blended shapes, where blending parameters may be manually tested, or in some instances, these blending parameters may be automatically computed if the user loads meshes or binary volumes representing an expected composite ablation zone per manufacturer data. Once commissioning is completed, operation by the ablation therapy software 32 resumes with the planning module 44. In one embodiment, the selection module 46 uses the commissioned data to precompute possible probe configurations from which a preferred solution or plan for treating a target region is selected. For instance, the selection module 46 may determine which of the blended ablation zones are to be selected or removed. As a simple illustration, and in the case of the selection module 46 comprising optimization functionality, an iterative solver of the selection module 46 may select a plan out of a plurality of (and in some embodiments, all possible) precomputed probe configurations (e.g., including single and/or multi-probe groups delivering a simple ellipsoid or blended ablation zones, respectively), such as a single probe delivering an ellipsoid ablation in one area of the target region and a triangular group of probes delivering a composite blended ablation at another area of the target region. In the case in which a grid template is used, the planning module 44 automatically computes a plurality of (and in some embodiments, all possible) insertion patterns and positions fitting or matching the grid openings/dimensions of the template, which would be an impractical endeavor for a user without the benefit of the ablation therapy software 32. Following the planning stage, operations resume with the guidance module 48 for guided physical placement of the probes, and then actual execution of ablation therapy via the ablation module 38 in conjunction with user control enabled via the GUI module 34.

Note that the memory 20 and storage device may each be referred to herein as a non-transitory, computer readable storage medium or the like.

Execution of the ablation therapy software 32 may be implemented by the one or more processors 16 under the management and/or control of the operating system 30.

When certain embodiments of the computing device 14 are implemented at least in part with software (including firmware), it should be noted that the ablation therapy software 32 can be stored on a variety of non-transitory computer-readable (storage) medium for use by, or in connection with, a variety of computer-related systems or methods. In the context of this document, a computer-readable medium may comprise an electronic, magnetic, optical, or other physical device or apparatus that may contain or store a computer program (e.g., executable code or instructions) for use by or in connection with a computer-related system or method. The software may be embedded in a variety of computer-readable mediums for use by, or in connection with, an instruction execution system, apparatus, or device, such as a computer-based system, processor-containing system, or other system that can fetch the instructions from the instruction execution system, apparatus, or device and execute the instructions.

When certain embodiments of the computing device 14 are implemented at least in part with hardware, such functionality may be implemented with any or a combination of the following technologies, which are all already existing in the art: a discrete logic circuit(s) having logic gates for implementing logic functions upon data signals, an application specific integrated circuit (ASIC) having appropriate combinational logic gates, a programmable gate array(s) (PGA), a field programmable gate array (FPGA), TPUs, GPUs, and/or other accelerators/co-processors, etc.

One having ordinary skill in the art should appreciate in the context of the present disclosure that the example computing device 14 is merely illustrative of one embodiment, and that some embodiments of computing devices may comprise fewer or additional components, and/or some of the functionality associated with the various components depicted in FIG. 2 may be combined, or further distributed among additional modules or computing devices, in some embodiments. It should be appreciated that certain well-known components of computer systems are omitted here to avoid obfuscating more relevant features of the computing device 14.

Before commencing further particulars of certain embodiments of the ablation therapy system 12 and the ability of the system to accurately represent complex composite ablation zones as well as enable improved interactions during execution of the ablation therapy plan, functionality of, or involving, the various modules of the ablation therapy software 32 are further described below. Beginning with the commissioning module 40, a brief description follows about certain requirements to fulfill when delivering a composite ablation zone as described on vendor datasheets. The definition or representation of composite ablation zones, also referred to as commissioning, prescribes the position and orientation of the applicators (or equivalently, probes) relative to the ablation zone, as well as the dimensions of the ablation zone. This commissioning may be performed as fixed (e.g., performed according to the manufacturer of the system) or configurable (e.g., performed by end-users of the system).

When configurable by the end-user, the system is equipped with a graphical user interface (GUI) (e.g., via GUI module 34, FIG. 2) that enables specifying the commissioning information, including the relative positioning of applicators and the composite ablation zone (e.g., tip offsets, spacing, etc.) and/or ablation zone dimensions (e.g., major and minor axes of an ellipsoid). The relative positioning of applicators and ablation zones can be expressed with respect to the center of the ablation zone or the tip location of any of the associated applicators. Further, the GUI is used to enable a user to virtually position a single probe or a group of probes that have been chosen during the commissioning phase, with feedback of the resulting ablation zones (e.g., composite or otherwise) presented on-the-fly to the user

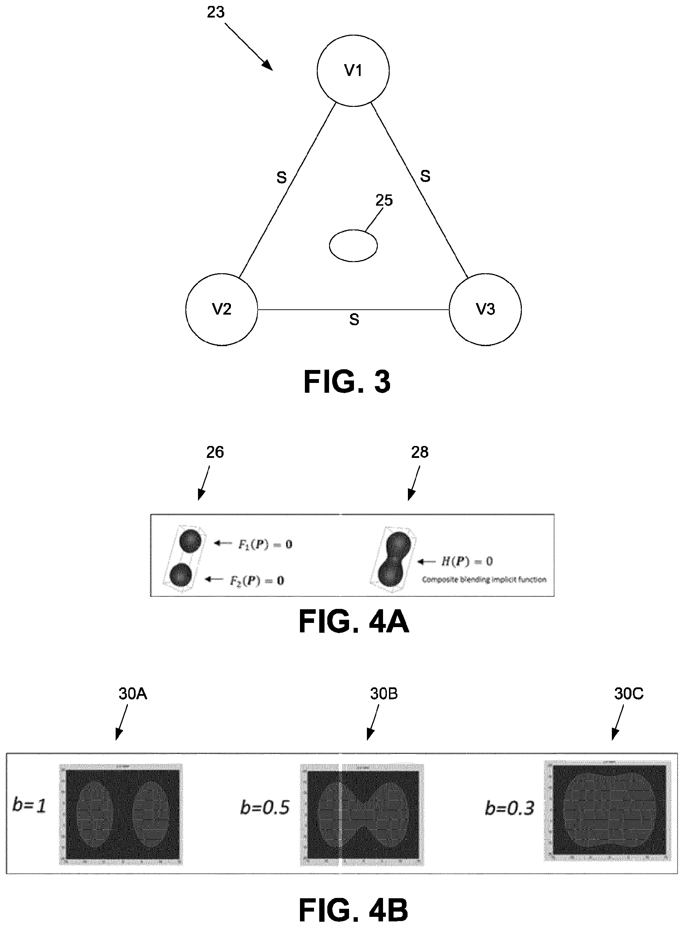

The user may be supported to specify composite ablation zones by a GUI (e.g., via GUI module 34 in conjunction with user interface(s) 24 as in FIG. 2) providing a CAD-like drawing environment enabling the relative positioning of applicators and composite ablation zone or a selection of pre-defined placement patterns, for which a limited number of parameters enables the relative positioning of applicators and composite ablation zone. The pre-defined placement patterns replicate information provided by the ablation device manufacturer, which includes, but is not limited to a line with applicators separated by distance, s (2 applicators), a triangle with sides of length s (3 applicators), a square with sides of length s (4 applicators), a trapezoid specified by a length and angle (4 applicators), etc. When the user selects one of these patterns, and provides values for the associated parameter(s), the system computes the relative positioning of the applicators with respect to the center of the ablation zone. For instance, and referring to FIG. 3, shown is a group of three applicators in the form of a triangle pattern 23. The three vertices V1, V2, and V3 represent respective applicator entry points (corresponding to entry trajectories), and the triangle pattern comprises a composite ablation zone entry trajectory 25 corresponding to the triangle barycentre. Parameter, s, indicates the length of each triangle side.

As an example, for an insertion pattern with an equilateral triangle shape of side s, the ablation entry trajectory origin may be positioned at (x,y) = (0,0) and the origins of the three applicator entry trajectories (i.e., triangle vertices V1, V2 and V3) may be obtained as follows:

$$V1(x,y) = \left( -\frac{s}{2}, -\frac{1}{3}\sqrt{S^2 - (\frac{S}{2})^2} \right) \qquad \text{EQN. 1}$$

$$V2(x,y) = \left( \frac{s}{2}, -\frac{1}{3}\sqrt{S^2 - (\frac{S}{2})^2} \right) \qquad \text{EQN. 2}$$

$$V3(x,y) = \left( 0, \frac{2}{3}\sqrt{S^2 - (\frac{S}{2})^2} \right) \qquad \text{EQN. 3}$$

As described above, certain embodiments of an ablation therapy system (e.g., ablation therapy system 12, FIG. 2) support not only ellipsoidal but also non-ellipsoidal composite ablation zones, such as those achieved by IRE, by providing a commissioning GUI (e.g., a GUI rendered on a display device, such as via GUI module 34 and user interface(s) 24 in conjunction with commissioning module 40, as in FIG. 2) that enables describing a composite ablation zone as a combination of ellipsoids along with an additional blending parameter (e.g., via composite model 42, as in FIG. 2). The individual ellipsoids are input to the composite model 42, where blending individual ellipsoids into a merged ablation zone is achieved. Notably, this blending is applied only between nearby applicators that are simultaneously activated to ablate tissue. As noted above, in some embodiments, the ablation therapy system 12 may use existing biophysical modeling techniques to represent the composite ablation zones.

The blending of individual ellipsoids into a composite ablation zone may be based on the mathematical concept of implicit functions, as provided via algorithms in composite model 42. An implicit function F(P) representing a surface specifies a position P to be on the surface if F(P) = 0. If the position is inside or outside the surface, F(P) < 0 or F(P) > 0, respectively. For an ellipsoidal surface around centroid Pc = (xc, yc, zc), the implicit function is defined by:

$$F(P) = F(x,y,z) = \frac{x-x_c}{r_x^2} + \frac{y-y_c}{r_y^2} + \frac{z-z_c}{r_z^2} - 1 \qquad \text{EQN. 4}$$

To calculate the composite ablation (for simultaneous ablation), the implicit functions of Ns individual ablation zones may be combined via another implicit function:

$$H(P) = 1 - \sum_{i=0}^{N_s} g(F_i(P)) = 0 \qquad \text{EQN: 5}$$

where g(x) is a blending function, and Fi(P) is the implicit function of the i-th surface.

As for the basic surfaces' implicit functions Fi(P), also for the combined implicit function H(P) it is valid that if H(P) = 0, the point P is on the surface, if H(P) < 0, the point P is inside the surface, and if H(P) > 0 the point P is outside the surface. FIG. 4A shows an example of two (ellipsoid) shapes 26 from implicit functions F1(P) and F2(P2), and an example of a blending implicit function, H(P) 28, originated from the implicit functions F1(P) and F2(P2).

The blending function g(x) can be defined in various ways, including, but not limited to:

$$g(x) = e^{-bx} \qquad \text{EQN: 6}$$

$$g(x) = \frac{a}{R^{n+1}}(x - R)^n(\frac{R}{a} - x)), \qquad \text{EQN: 7}$$

where b, a, R, and n are blending parameters, and where these two blending functions are used to enforce a smooth transition between two basic surfaces (e.g., two ellipsoids). The exponential blending function of equation 6 comprises parameter b, where b > 0 is a parameter to control the blending curve steepness (e.g., where b = 1 comprises two separated ellipses, b = 0.5 comprises slight blending, somewhat similar to an hourglass shape, and b = 0.3 comprises more extensive blending where the overall shape is more oblong with a slightly narrower middle region), as illustrated by the blended implicit functions H(P) 30 (e.g., 30A, 30B, and 30C) in FIG. 4B.

Equation 7 comprises a distance-based blending function, where the function is true if $x \leq R$, otherwise, $g(x) = 0$. In equation 7, $a \in [0,1]$, $n \in N^*$, and $R > 0$ is a blending radius. Here, the blending action vanishes when $x > R$, and parameter, $n$, adjusts the blending function by steps and parameter, $a$, realizes a fine-tuning in between the steps. The value(s) for the blending parameter(s) may be set directly by a value provided by the user, or indirectly by automatic adjustment of the blending parameter to mimic a composite ablation shape prescribed by a mesh provided by the user. For instance, the user may change one or more of the blending parameter values, and the resulting blended shape may be computed on-the-fly and the commissioning module 40 may, in cooperation with the GUI module 34, provide visual feedback of the newly blended shape. In some embodiments, the computed shape may be plotted next to the shape proposed by the manufacturer. In some embodiments, quality metrics may be provided (e.g., Dice coefficient) to show how the current blended shape overlaps with a reference shape. As to the mesh-based value determination approach, in instances where an ablation device manufacturer computes an expected composite ablation shape, such as via the use of bio-heat transfer modeling the probes and the anatomy, these computed composite ablation regions may be provided as meshes or binary masks. In such instances, all blending function parameters may be inferred using, say, an I-squared optimization method or other methods that minimize or reduce the error between the computed meshes and a reference ground truth mesh provided by the manufacturer.

The value(s) for the composite ablation modeling parameters, or blending parameter(s) from the above mathematical equations, may be set as a fixed constant during development, as a configurable constant to be configured by the user, as a variable parameter controlled by the user on a case-by-case basis, or as a calculated parameter controlled by the system on a case-by-case basis.

In embodiments where the composite ablation modeling parameter(s) are constants to be configured by the user, the user interface (e.g., via GUI module 34) may combine any numerical input with example renderings of the composite ablation modeling result (e.g., blended ablation zones). This user interface may enable configuring the composite ablation modeling parameter(s) globally, as accessible through a system configuration user interface, per user, as accessible through a user preferences user interface, or per ablation device/zone, accessible through an ablation device configuration/commissioning user interface.

In embodiments where the composite ablation modeling parameter(s) are variable and controlled by the user on a case-by-case basis, the user interface may include (but is not limited to) any one or a combination of user interface elements that include one or more slider controls connected with the composite ablation modeling parameter(s), one or more pull-down menus with pre-set values for the composite ablation modeling parameter(s), direct mouse manipulation (e.g., left or right-mouse drag) connected with the composite ablation modeling parameter(s), or a scroll wheel connected with the composite ablation modeling parameter(s).

In embodiments where the composite ablation modeling parameter(s) are calculated by the system on a case-by-case basis (where reference to calculation may range anywhere from applying potentially complex mathematical relations to use of simple look-up tables), the resulting, calculated value may be determined from one or any combination of the positioning of applicators relative to each other, the positioning of applicators relative to patient anatomy, the time used to ablate simultaneously, configurations of the ablation device (e.g., if a parameter is configured per ablation zone), or settings of the ablation device (power, frequency, etc.). As an example of criteria the system may use to arrive at parameter values, given a set of positioned applicators, the system may store an internal bioheat transfer equation model able to predict the ablated region, and this region may be converted to a binary mask (e.g., a computed blended composite ablation zone).

Having described certain aspects of commissioning (e.g., via commissioning module 40 and composite model 42, FIG. 2), attention is now directed to the planning module 44, including the selection module 46. Certain embodiments of an ablation therapy system 12 include an automatic planning component (e.g., via planning module 44, FIG. 2) to support a user (e.g., clinical expert) in establishing an ablation plan. The thermal ablation planning selection problem generally consists of a preferred (e.g., depending on one or more user criteria), or in some embodiments, an optimal selection of a set of applicators delivering single/regular and/or composite ablation zones to ablate the target region (e.g., tumor plus an optional margin) as well as possible (e.g., at 100% of its volume) while, at the same time, sparing nearby organs at risk and normal tissue. The automatic planning features of the planning module 44 may be implemented to support planning of composite ablations, as well as the planning of regular ablation zones achieved using a single applicator, for grid-based and free-hand ablation procedures. When a composite ablation zone is commissioned and used for a given treatment, multiple applicators are to be inserted in the subject's body according the defined placement pattern, and activated simultaneously to achieve the composite ablation zone within the target region. The delivered composite ablation zone resides along an entry trajectory that lies in-between the entry trajectories used for the insertion of the applicators. Usually, this so-called composite ablation entry trajectory traverses through the center of the composite ablation zone, which coincides with the barycentre of the corresponding applicators. This composite ablation entry trajectory is oriented parallel to the direction of the corresponding applicators.

Certain embodiments of an ablation therapy system 12 comprises an automatic planning component of the planning module 44 that supports composite ablation zones, and in particular, uses a group of entry trajectories that are computed for a composite ablation zone during commissioning, including the insertion paths used for positioning both the medical applicators and the corresponding composite ablation zone. More specifically, the ablation therapy system 12 disclosed herein defines (and improves over) pre-determined groups of applicators with different placement patterns, which are executable via a plurality (and potentially, all) possible grid holes (or for grid-less, via skin entry points, on virtual planes orthogonal to user provided directions).

In general, a collective of plural groups of entry trajectories based on repeated patterns (e.g., triangles) or a mix of patterns (e.g., single ellipsoid, linear two-applicator-based patterns, etc.) according to single and multi-probe ablations may be part of an ablation therapy plan. These multiple patterns of applicators are arranged within one or more bounding boxes. As similarly described in association with FIG. 3, the vertices of a pattern are the entry points used by the applicators. The ablation zone entry origin lies along the barycenter of this pattern. The vertices of these groups are considered collectively as a unique entity, delivered via a given geometrical placement pattern, and the group of applicators (e.g., such as in a triangle pattern, or square pattern, etc.) delivers a blended composite ablation zone, where the ellipsoid zones of the vertices are blended to form the composite ablation zone shape. In some embodiments, collective shifts (e.g., the groups within the boundary as a collective single entity) in the x-direction, y-direction, and/or rotational shifts may be achieved to offer improved coverage of the targeted region. Given a set of user insertion directions, groups of entry trajectories may be shifted (e.g., in the x and/or y direction and/or rotational shifts) within bounding boxes encompassing 2D target projections computed over virtual planes perpendicular to the user directions.

Based on manufacturer data and the commissioned single and/or composite ablation zones selected by the user for treatment (e.g., using different device settings to deliver simple and/or complex ablation zones, such as via linear patterns, triangle patterns, rectangle patterns, etc.), entry patterns of a group of probes and the relative blending ablation zone for the group is precomputed. This process may involve the translation/rotations described above. Given the precomputed set of a plurality of blended ablation zones delivered by the groups of probes entered via multiple sampled entry points, and with tips positioned at multiple and different depths, processing resumes via the planning module 44. In the planning module 44, in one embodiment, the selection module 46 comprises a set-covering, greedy, iterative optimizer that selects a preferred set of probe groups (e.g., triangle patterns in this instance, though the preferred set in some scenarios may include different shapes, including triangles, squares, dots, etc.) to cover the tumor (target region) while sparing the risk organs nearby. In effect, the final treatment plan is the combination of the multiple probes' patterns of grouped applicators delivered over a single or multiple bounding boxes orthogonal to a single or multiple directions provided by the user.

Note that selection of the preferred set may be an optimized or best set in some embodiments, or a set that is less than what may be considered as the best set yet suitably meets certain criteria. For instance, example criteria may include quantity or computational time of iterations in selection, anticipated time of treatment, the quantity, size, and/or maturity of the tumors, assessed or predicted risk of malignancy, confidence in such an assessment or prediction, subject and/or subject family history, among other factors established in the medical community and/or by the clinical expert. One or more of these factors may be considered and/or weighted relative to one or more other factors in a manner that might result in an outcome that is less than the best outcome as achieved by an optimizing function, yet preferred based on the established criteria. In some embodiments, a preferred solution may comprise greater subjectivity in considering an outcome than an optimization function, and/or in some embodiments, an optimizing function may be used yet constrained based on computational resources and/or computation time.

Note that in some embodiments, multiple bounding boxes that are orthogonal to the user-given entry directions may be used, and the shifts (translational or rotational) may be performed independently (e.g., an x-direction shift alone or in combination with a y-direction shift and/or rotational shift, a y-directional shift alone or in a combination with one or more other shifts, etc.). Further, it is noted that the bounding box is a generalized concept for both grid- and grid-less scenarios. In the grid case, the bounding box is given by the grid template geometry, and in the grid-less case, the bounding box is virtually computed over a plane orthogonal to the user given directions.

In a grid scenario, the sampling of groups of entry trajectories is strongly constrained by the regular grid geometry and the grid holes sampling value. For instance, an equilateral triangular pattern cannot be delivered via the grid templates currently supported by some systems, since an equilateral triangle has 60-degree angles, while a square grid has hole-hole diagonals at 45-degree angles. Differently, groups of 2-, 3- or 4-applicators with a linear/segment pattern may be delivered via a grid template if the relative distance of the applicators is a multiple of the grid holes spacing. A simple and straight-forward way to determine placement pattern positions matching the grid holes of a grid or grid template, where the linear patterns match the grid hole spacing dimensions, may be implemented in planning. For instance, entry trajectories based on x-direction and/or y-direction iterative shifts for a two-applicator linear pattern in a grid usage scenario may be used, where shifts according to grid spacing or a multiple of the grid hole spacing occur iteratively for columns and rows of the template grid. In contrast to existing approaches, the ablation therapy software 32 (FIG. 2) automatically computes a plurality of (or potentially all of) the groups of probes that match the grid spacing, and takes into account the blended composite ablation zones during selection. Existing techniques only consider each probe in isolation, and do not propose a mechanism to precompute the plural geometrical groups of probes matching the grid geometry (and do not consider blended shapes during selection).

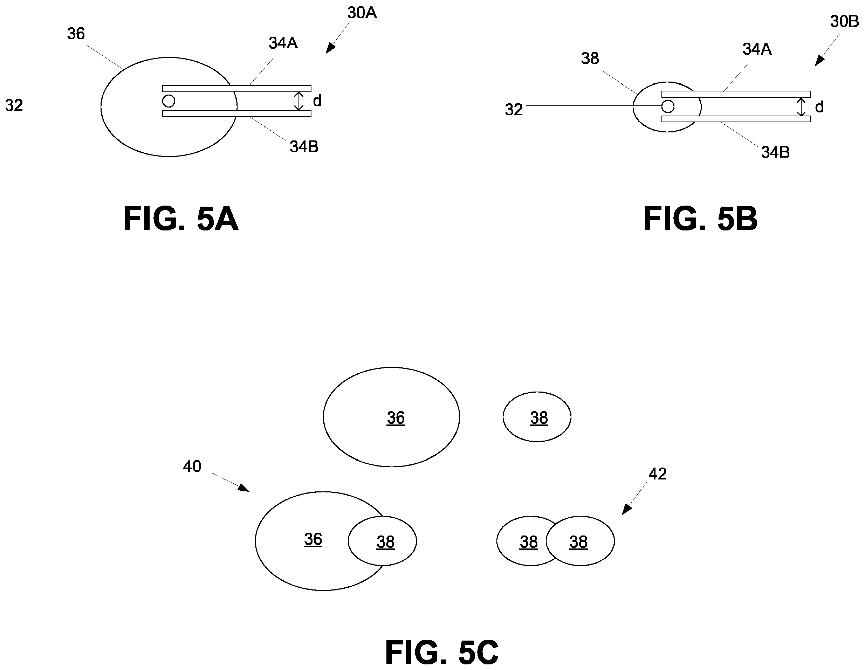

FIGS. 5A-5B show ablation shapes for a two-applicator linear (placement) pattern, which includes a type of information that may be referenced in a device datasheet and stored by the ablation module 38 (FIG. 2). In this particular example, and referring to FIG. 5A, shown is an applicator set 30A comprising applicators 34A, 34B that correspond to applicator trajectories, and further shown is the ablation trajectory 32 for this particular set 30A. Applicator separation dimension, d, may be applicable to the given applicator set, and in one example, may be 1.0 cm. FIG. 5B shows an applicator set or group 30B comprising applicators 34A, 34B that correspond to applicator trajectories, and further shown is the ablation trajectory 32 for this particular set 30B. Notably, the patterns in FIGS. 5A and 5B deliver two different composite ablation zones 36 and 38, respectively, for two different device settings (e.g., power and time ON or duration at the given probe separation dimension). For instance, ablation shape 36 may be based on a higher power level provided via applicator set 30A when compared to the ablation shape 38 generated by applicator set 30B, with dimension d having a value of, for instance, 1.0 cm. In other words, applicator sets 30A and 30B may be similarly dimensioned though used at a different power and/or duration level to provide a different composite ablation shape/size.

FIG. 5C shows the resulting composite ablation zones based on differences in settings and also combinations of composite ablation zones 40 and 42 as selected by the optimization module 46 (FIG. 2) to plan ablation therapy treatment. That is, aside from the individual composite ablation zones 36 and 38, additional groups of applicators using the same and/or different placement patterns may be combined by the optimization module 46 to provide for further coverage, such as shown with the combined composite ablation zones 36 and 38. Though depicted in FIGS. 5A-5C using a combination of simple composite ablation zones 36 and 38 (e.g., shaped respectively as ellipses), the choice of ellipses as a shape is merely for illustration, and as should be appreciated, the group composite ablation zones may be ellipsoidal or non-ellipsoidal (e.g., blended shapes), such as shown in FIGS. 1A-1C. In some embodiments, the selection module 46 may take into account this geometrical information about the probe group(s) and the composite ablation zone when selecting a preferred plan (based on selection among the commissioned probes) covering a tumoral region.

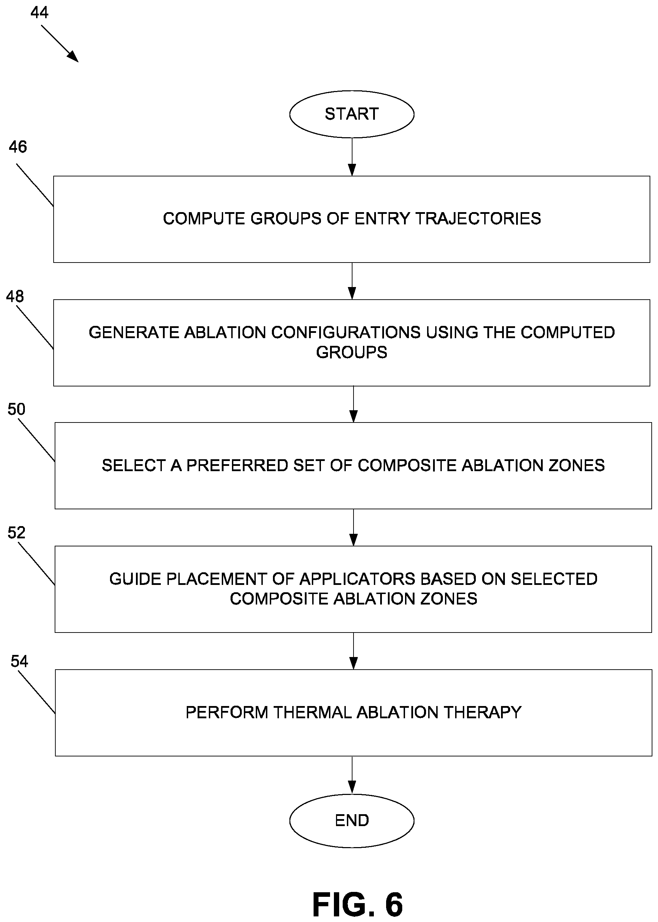

In view of the description above, it should be appreciated that certain embodiments of an ablation therapy system (e.g., ablation therapy system 12, FIG. 2) provide for ablation configuration generation, as described above, and automatic planning and selection. FIG. 6 shows an embodiment of an example automated planning method 44, which may be implemented by the ablation therapy system 12. As described above, the method 44 computes groups of entry trajectories (46). The groups may be of the same pattern, or a mix of patterns. Shifts in the x and/or y direction, and/or roto-translational shifts may be used, as explained above. For grid applications, shifts according to grid hole spacing or multiples thereof in the x and/or y direction may be used. The method 44 further comprises generating ablation configurations using the computed groups (48). The final set of sampled groups of entry trajectories belonging to commissioned composite ablation zones are used to generate ablation configurations (e.g., a set of one or more partially overlapping ablation zones lying along the groups' barycentre entry trajectory and covering the segmented target regions). In each case, differences in settings may be used to achieve composite ablations. Note that the total set of ablation configurations covering the target region may be a combination of simple and composite ablation zones.

The method 44 further comprises selecting a preferred set of composite ablations (50). In an optimization embodiment for selection functionality, the huge set of generated ablation configurations are passed as an input to a heuristic iterative set covering optimization algorithm (e.g., via selection module 46, FIG. 2), which selects the preferred (e.g., minimum) set of composite (and simple) ablation zones covering the target region as much as possible, while sparing the risk organs nearby. Here, the ablation zones configuration is processed to see how well it covers the target area and spares the risk organ regions. To do this, in one embodiment, a set of ablation coverage volume-based quadratic functional objectives may be used, as disclosed in commonly-assigned international publication WO2021/032449.

Digressing briefly, for grid-based applications, the heuristic iterative discrete optimization method described in WO2021/032449 may be extended to support the manual positioning of the applicators using grid holes that respect a minimum distance requested by the user, or prescribed by the system itself, or other entities (e.g. applicator vendor). Hereto, firstly, the user is asked to provide a given minimum grid holes' (or probes') distance value. Secondly, when an optimal applicator configuration is selected at each inverse planning optimization iteration, all grid holes within a given distance from the currently selected hole are inactivated and made unavailable to the mathematical solver over subsequent optimization iterations. Finally, the computed optimal set of ablation probes are visualized on a suitable GUI. Accordingly, an iterative ablation optimization algorithm as extended from that disclosed in WO2021/032449 is able to enforce a threshold (e.g., minimum) distance between finally selected single applicators and ablation zones to deliver for treatment, which allows the work of the clinical expert (user) to be simplified by avoiding potential device collisions that could hamper the exact treatment execution.

The above-described idea from WO2021/032449 may be reused in method 44 for composite zones that correspond to groups of applicators as described herein. When groups of applicators are commissioned and used for ablation therapy treatment, the threshold distance between applicators is replaced with (replaced by) a more general threshold distance between groups of applicators, which is defined as the threshold (e.g., minimum or user or system-defined) distance between pairs of applicators belonging to the same and/or different groups (e.g., for composite ablation zones). For instance, a minimum distance d may be enforced among pairs of delivered applicators. Assuming two groups of applicators (e.g., one with a triangular placement pattern and the other with a linear placement pattern) are delivered, enforcement includes ensuring that the minimum distance d is enforced over the pairs of applicators, without any distinction if they belong to different or the same group.

During the optimization iterations of the above-described embodiment, a solver iteratively picks an optimal ablation configuration from the big set of ablation configurations created at the previous steps discussed above. Hereto, potential configurations associated to groups of applicators positioned too close to the currently selected optimal set (e.g., groups of applicators are closer than a minimum distance provided by the user) are neglected and not selected.

Alternatively, or in addition to the threshold distance between groups of applicators, another requirement may be to avoid or reduce overlaps between group patterns (e.g., having two triangular groups partially overlapping). This event may make the treatment execution too complex, and the newly introduced threshold distance between applicators groups may not be enough to avoid such a situation. A potential solution to avoid or reduce such overlap between groups of applicators may be to develop the "point-in-polygon" check method. Given two groups of applicators, for instance, two sets of triangular insertion patterns (each as shown in FIG. 3), a check may be made to assess if any of the vertices (e.g., applicator insertion points) of one triangle lies inside the other one, or not. If the check identifies an overlapping pattern, a current ablation configuration belonging to the current groups of applicators is neglected and not processed further by the optimization algorithm.

It is noted that while placing applicators inside the pattern of another composite ablation zone is not desired, re-using applicators positioned to deliver one composite ablation zone to deliver another composite ablation zone may be useful in certain clinical usage scenarios. Accordingly, some embodiments of method 44 may enable such multiplicity, re-using of virtual applicators to contribute to multiple composite ablation zones.

Though the method step 50 is described primarily above in the context of an optimization method, it should be appreciated that a preferred selection may be achieved based on a truncated optimization and/or one or more criteria as described above, where the preferred selection may be different than a fully-performed, optimization-driven result.

In some embodiments of an ablation therapy system (e.g., ablation therapy system 12, FIG. 2), interactive, manual planning of composite ablations may be supported by providing the user with the ability to either virtually position composite ablation zones and derive associated applicator positioning, or virtually position applicators and derive a composite ablation zone if possible. For instance, the user may position some groups of probes and select the composite ablation zone to deliver treatment (e.g., produced a commissioned (based on power, time) pair to use). This positioned, blended composite ablation zone may be used to initialize the iterative selection (e.g., from step 50 in FIG. 6). In some embodiments, the composite ablation zone may already cover some parts of the target region, so selection functionality focuses on selecting new probe groups to cover the remaining uncovered parts. In some embodiments, the user may commission a number of devices with different placement patterns, which may be provided in a GUI for user selection. In some embodiments, the GUI may trigger an error indication when an unfeasible pattern is selected not matching the grid geometry. In one embodiment, the user may position a composite ablation zone using a given user interface. In some embodiments, the above-described deriving of applicator positioning or composite ablation zones may be automated based on the user's initial input, as described above. Here, the corresponding group of applicators (e.g., with a triangular insertion pattern) may be positioned (by the system) and adjusted (e.g., rotated around the composite ablation zone insertion axis) by the user using suitable user interface tools (e.g., a virtual or physical scroll wheel, etc.). For example, the user may change the ablation shape (e.g., via adjustment in power/duration settings) to deliver over a positioned group, and/or the user may rotate, when possible, the group placement around the composite ablation zone axis.

In some embodiments, after the user positions a composite ablation zone using a given user interface, a dedicated selection process (e.g., an automatic optimization loop) may be executed to search a preferred (e.g., an optimal) rotation angle to use for the positioning of the corresponding group of applicators.

In embodiments where the user is enabled to virtually position individual applicators, an associated composite ablation zone may be proposed by the system. The user may have control of selecting different composite ablation zones that match the current positioning of virtual applicators (e.g., change the ablation zone shape via a change in settings).

In some embodiments, the user may be guided in the virtual positioning of subsequent applicators based on the potential composite ablation zones to be used using the same or different patterns (or in the case of grid-less treatment, over the same or different virtual boxes). For instance, once a first applicator is virtually positioned, only a few virtual locations may be used to achieve the commissioned composite ablation zones. These few virtual locations may be visualized using overlay graphics.

Note that one or more of these interactive planning methods may be used to plan complete ablation procedures (i.e., from scratch), as well as to correct/adjust ablation plans calculated by the automated planning component.

Method 44 further comprises guiding (e.g., via guidance module 48) placement of applicators based on selected composite ablation zones (52). Applicator placement may be guided by a display of overlay graphics on top of per-operative medical imaging, including, but not limited to, ultrasound imaging (e.g., 2D/3D in real-time), fluoroscopy (real-time), magnetic resonance (e.g., MR, in 2D/3D real-time or static), computed tomography (e.g., CT (3D static)), or cone beam CT (e.g., CBCT (3D static)). In addition, the per-operative imaging may be complemented with fusion of pre-operative imaging to visualize otherwise invisible target regions. Such pre-operative imaging may include, but is not limited to, MR, CT, single photon emission CT (SPECT), or positron emission tomography (PET). Applicator placement may not be according to plan in some instances, and so the ablation therapy system includes a specified behavior for dealing with placement inaccuracy. In one embodiment, in case placement deviates from the plan, the system may raise an alarm based on a fixed threshold for placement error, raise an alarm based on a configurable threshold for placement error, or have no alarm. In addition, the system may still visualize an anticipated ablation zone relative to the average tip location and orientation, an anticipated ablation zone according the initial plan (i.e., remains unchanged), or may not visualize an anticipated ablation zone.

Guidance may further be complemented with adaptive plan improvements, effectively re-computing the selected plan for the remainder of the procedure given already placed or performed ablations. In case only some of the applicators associated with a composite ablation zone are placed, and others are planned, adaptive planning may re-select the composite ablation using the (average) orientation and depth of the placed applicator(s), or keep the composite ablation as is, and only select positioning of applicators associated with other ablation zones.

Finally, the method 44 comprises performing ablation therapy based on the selected composite ablation zones (54), which is the phase after planning (e.g., plan execution/implementation). Once the plan is selected, certain embodiments of the ablation therapy system 12 represent all ablations to be delivered in such a way that the user knows what is to be activated sequentially and what is to be activated simultaneously. In implementing the ablation plan, the ablation therapy system 12 may include one or more graphical user interfaces to assist the user in monitoring and controlling the activation of applicators (e.g., sequential or simultaneous activation). For instance, the GUI module 34, in cooperation with the ablation module 38 and based on the ablation plan provided by the planning module 40, provide a visual mechanism to distinguish between sequential and simultaneous ablations to be executed, and further provide the user the ability to change the activation modality of groups of probes/applicators (when feasible). Stated otherwise, the user is provided the ability to easily distinguish between single (ellipsoidal) ablations that should be delivered sequentially, and (group) of (blended) ellipsoidal ablations that should be delivered simultaneously.

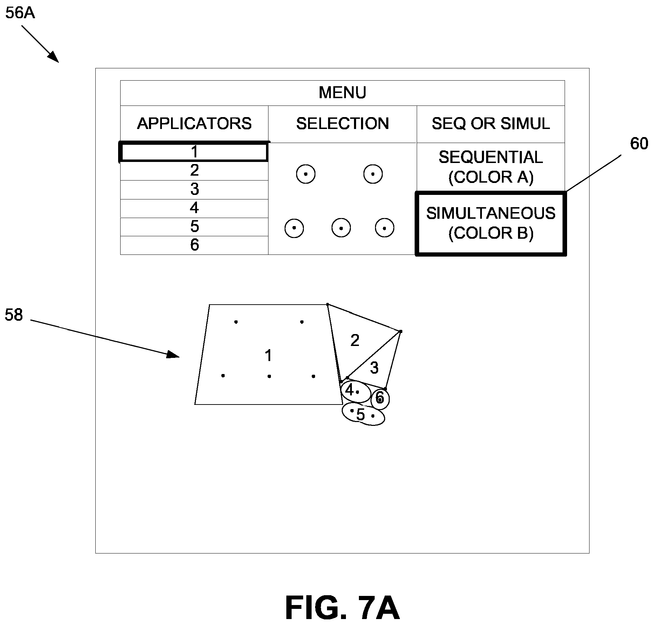

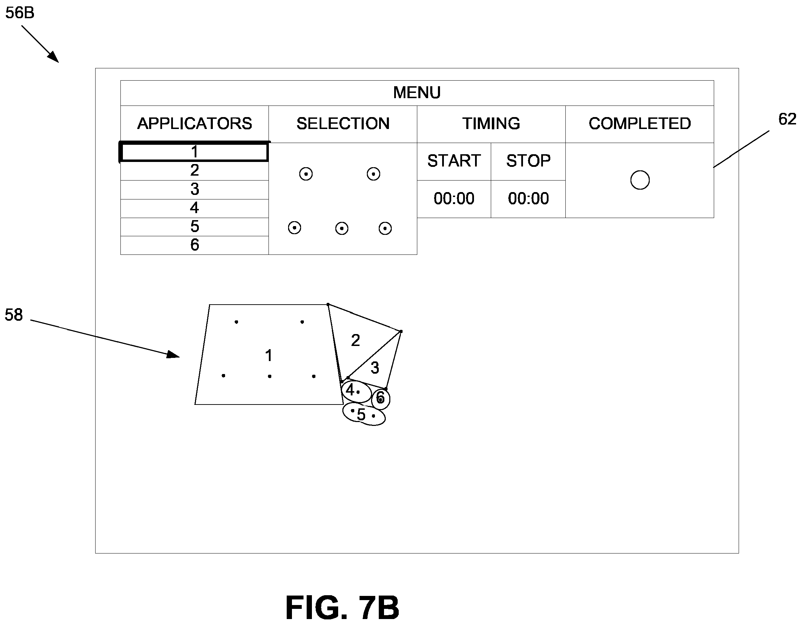

Accordingly, the ablation therapy plans are represented in a way that the user can distinguish simultaneous and sequential ablation, which may be achieved by grouping simultaneous ablations into one and applying visual distinctions (e.g., color-coding, shading, labels, or other markings) for simultaneous ablations. To keep the clinical user in control of the ablation plan, the graphical user interface(s) may include one or more tools to adjust whether certain ablations are performed simultaneously or sequentially. Indeed, correct application of the blended composite ablation modeling (and more advanced biophysical models alike) benefits from a distinction between simultaneous and sequential ablation when implementing the plan. FIGS. 7A and 7B are example screen diagrams that illustrate an embodiment of example graphical user interfaces that enable visual distinctions for sequentially and simultaneously activated applicators for an ablation therapy plan and for controlling ablations. Referring to FIG. 7A, shown is an example GUI 56A that may be rendered on a display device (e.g., UI 24, FIG. 2) via GUI module 34. The GUI 56A comprises a representation of an ablation plan (e.g., ablation therapy plan) 58 provided by the planning module 44, which includes plural ablation zones (e.g., ablation zones 1 - 6) corresponding to plural applicators (trajectories denoted by black dots) that are used to treat a target region (e.g., a tumor with an optional margin). The plural ablation zones in this depicted example comprise a mix of single and composite ablation zones. For instance, in this example, composite ablation zones consist of zones 1, 2, 3, and 6, and single ablation zones consist of zones 4 and 5. It should be appreciated that the ablation plan representation 58 shown in FIG. 7A is merely an illustrative example, and that other shapes of zones and/or quantities of applicators may be used.

Also shown is a user interface tool 60 that enables a user to select the applicators for respective ablation zones 1-6 and to select the color coding of each of the ablation zones of the ablation plan via selection of the activation type (sequential or simultaneous) for the selected applicators. The tool 60 may be prompted via selectable options in a (top) banner menu, or other ways (e.g., clicking on the ablation zone representation 58, selecting a short key on a keyboard, etc.). In the depicted example, the user may select applicators for zone 1 (as depicted by the highlighted box labeled 1 in the first or leftmost column of the tool 60), which may prompt a depiction of the corresponding applicator group pattern in the window in the second or middle column of the tool 60. In one embodiment, the applicators are automatically selected based on the ablation plan indicating the selected ablation zone 1 is a composite ablation zone. In some embodiments, individual applicators for the zone may be selected (e.g., providing flexibility of the user to alter the initial ablation plan). Applicators may be de-selected (e.g., based on a user deciding to change the ablation plan), such as by selecting one or more of the applicators presented in the selection window (e.g., using a mouse). Errors in selection (e.g., to change the ablation plan) may be prompted as an indicator, such as a warning label or symbol or error message, in the GUI 56A after processing the selections and determining the selections as simultaneously activated or sequentially activated to be prohibited. For instance, the user may select only a single applicator for simultaneous activation, which after processing (e.g., the last column selection, or after all ablation zones are completed), may prompt the error message. The user may select a single applicator via cursor selection, and groups of applicators by using select+Ctrl->Shift->arrow keys, select-and-drag manipulations with a mouse, among other methods.

The third or right-most column of the tool 60 comprises a selection option for sequential activation for the selected applicators of the selected ablation zone, or simultaneous activation. Selection of one or the other in this right-most column results in the corresponding selection of one of two colors for the particular selected zone (color A for sequential, color B for simultaneous). Colors A and B are visually distinguishable, and may be any of a plurality of different colors (e.g., color A may be green, color B may be orange). In some embodiments, the color choices may be preconfigured, or in some embodiments, configurable by a user. In some embodiments, if there is no change in plan via selection entries, the activation options may be automatically selected based on the ablation plan. In this example, the user observes the applicators of ablation zone 1 to all be selected, and assuming no change in plans by the user (e.g., deselecting one or more of the applicators in the selection column, such as via mouse-click), selects the simultaneous selection in the right-most column to enable the applicators to be simultaneously activated during implementation of the ablation plan and for the corresponding zone (e.g., ablation zone 1) to be of color B. After ablation zones and corresponding applicators are chosen and associated with either sequential or simultaneous activation, the ablation plan representation 58 is modified (e.g., on-the-fly) to show the color changes. When selections are completed, the simultaneously activated ablation zones are logically (and visually) grouped together.

In some embodiments, other visual distinctions may be used to distinguish between simultaneous and sequential activations, such as differences in shading, hatching, labels, etc. In some embodiments, the user may be able to make some selections on either the tool 60, the ablation plan representation 58, or both. For instance, the user may select the applicators for ablation zone 1 in the leftmost column of the tool 60, and the applicators are highlighted in the ablation plan representation 58. The user may then select from one of the two choices in the right-most column in the tool 60. As another example, the user may simply make the activation choice on the ablation plan representation 58 by toggling on the ablation zone (e.g., clicking once for color A, and again for color B). These and other mechanisms for making selections via a graphical user interface may be implemented as alternatives to the description above, and hence are contemplated to be within the scope of the disclosure.

In general, during the planning (including optimization) phase, the returned ablation plan contains a feasible set of sequential and simultaneous groups of ablations. However, during the implementation phase, the user may manually change the state of an ablation or of a group of ablations. In this case, the expected ablation zone is recomputed, which may or may not use the composite ablation model to compute the blended ablation zone of a simultaneous group of ablations. The ablation therapy system 12 may check whether an unfeasible situation occurs. For instance, the user may enforce, by mistake, simultaneous pull-back ablations along the same trajectory at different depth, or the user may manually generate a simultaneous groups of ablations for an applicator that was not commissioned by the user during the commissioning phase. Explaining further, should the user decide to change plans (e.g., deselect one or more of the applicators, or alternatively for embodiments where applicators are not automatically selected, select only a subset of the applicators shown in the selection window), the ablation plan 58 is altered on-the-fly to provide the user with feedback of the change in ablation coverage. In some embodiments, attempts to change the ablation plan may be prohibited, where a warning message or flag or other symbol may be prompted on the GUI 56A to alert the user that the change is not feasible. Explaining further, the ablation therapy system 12 may operate as a smart-system, prohibiting simultaneous ablation and associated composite ablation modeling where necessary or based on feasibility. For example, a plan for a pull-back ablation indicates two locations along a single trajectory for a particular applicator. Consequently, it is impossible to simultaneously ablate on both these locations, and any attempt by the user to configure the ablation in this manner is prohibited (with in some embodiments, a corresponding warning or error message). Another situation in which application of composite modeling includes selecting more applicators to be used simultaneously than is supported by the ablation device (e.g., selecting five (5) cryo-applicators while the system offers only four (4) channels). Note that one or more of the user-interactive features disclosed herein during the implementation stage of the ablation plan may also be used in earlier stages, such as the planning stage.

As long as the sequential and simultaneous groupings are not changed, the ablation therapy system 12 may autonomously determine the sequence of activation. That is, the ablation therapy system 12 may simply order the zone activations for execution without user intervention. However, the user is free to decide a different activation sequence, if needed. Referring to FIG. 7B, shown is the GUI 56B with the ablation plan representation 58, and another tool 62 that enables the user to control and track progress of the ablation plan implementation (e.g., start and stops, marking groups where ablation is completed, etc.). The GUI 56B may automatically be prompted after completion of steps in GUI 56A, or invoked via selection of an option in the menu. In this depicted embodiment, the ablation plan representation 58 comprises ablation zones 1-6 grouped and colored according to their respective activation status, and the user can start and stop ablations for each ablation zone via the timing column, and mark an ablation as completed (or in some embodiments, also stopped). After applicator placement and during the monitoring of the actual ablation process, the GUI 56B may enable a user to indicate simultaneous ablation and/or control by marking a planned group of ablations as completed/ablated, starting and/or stopping a planned group of ablations, marking a selection of multiple ablations as completed/ablated, starting and/or stopping a selection of multiple ablations, starting and/or stopping individual ablations within a short time span (e.g., two 3-minute ablations started or stopped within five (5) seconds of each other, etc.). In some embodiments, particularly for changes in the ablation therapy plan, the GUI 56B or 56A may enable modification of the blending parameter(s). Note that reference to starting and/or stopping refers to the fact that ablations may be considered to be performed simultaneously and the composite ablation modeling may be applied when ablations are started and stopped at the same time, ablations are started at the same time, or ablations are stopped at the same time. In some embodiments, the GUI 56B may present an indication to the user when each of the plural applicators is activated (e.g., via a pop-up alert, or highlight around a given ablation zone, etc.).

Note that in some embodiments, particular values for the composite ablation modeling parameters may be used to implicitly define whether ablations are to be modeled as sequentially or simultaneously activated (e.g., if a particular blending factor is set to zero, the individual ablations are not blended during composite ablation modeling determinations, thus indicating sequential ablation). For instance, in case of a blending parameter equal to zero, the blended composite shape will be identical to the 3, 4 or whatever quantity of ellipsoids are delivered without blending. Since such ablations are not blended, these ablation zones should be shown to the user on the GUI 56A or 56B as sequential ablations.

In embodiments where the parameters of the composite ablation modeling are automatically applied based on the time ablations are performed simultaneously, users should be able to indicate the exact time of activation and deactivation for a particular applicator, since a further integration between the ablation and guidance systems is preferred to prevent error. This integration should enable the guidance system to be notified whenever an applicator is activated with the ablation system. Note that this applies to advanced biophysical models that are capable of modeling the impact of activation and deactivation delays between the individual ablations.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. Note that various combinations of the disclosed embodiments may be used, and hence reference to an embodiment or one embodiment is not meant to exclude features from that embodiment from use with features from other embodiments. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical medium or solid-state medium supplied together with or as part of other hardware, but may also be distributed in other forms. Any reference signs in the claims should be not construed as limiting the scope.

**Claims**

1.   A system (12), comprising:

a display device (24);
a memory (20) comprising instructions (32); and
one or more processors (16) configured by the instructions to:

receive an ablation plan comprising plural ablation zones corresponding to plural applicators, the plural ablation zones comprising one or more single ablation zones and one or more composite ablation zones; and
provide on the display device a representation of the ablation plan (58) and a visual distinction between sequentially activated applicators and simultaneously activated applicators, the plural applicators comprising the sequentially activated and simultaneously activated applicators.

2.   The system of the preceding claim, wherein the one or more processors are further configured by the instructions to provide the visual distinction based on color coding.

3.   The system of any one of the preceding claims, wherein the sequentially activated applicators provide single ablations and the simultaneously activated applicators provide composite ablations, wherein the composite ablations are based on blending implicit functions.

4.   The system of any one of the preceding claims, wherein the one or more processors are further configured by the instructions to make an adjustment to one or more of the plural ablation zones based on a change in state of the ablation plan, wherein the adjustment comprises one or a combination of recomputation of the one or more of the composite ablation zones or the one or more of the single ablation zones.

5. The system of any one of the preceding claims, wherein the one or more processors are further configured by the instructions to prohibit user-requested adjustments to the ablation plan that are unfeasible to implement.

6. The system of claim 5, wherein the user-requested adjustments include simultaneously activated applicators for pull-back ablations, an ablation based on an uncommissioned applicator, or simultaneously activated applicators that are greater than a number of available channels.

7. The system of any one of the preceding claims, wherein the one or more processors are further configured by the instructions to provide a graphical user interface (56A, 56B) that enables a user to control ablations corresponding to the plural ablation zones of the ablation plan.

8. The system of claim 7, wherein the graphical user interface enables a user to perform one or any combination of the following:

mark a planned group of ablations as completed or ablated;
start and stop a planned group of ablations;
mark a selection of multiple ablations as completed or ablated;
start and stop a selection of multiple ablations;
start and stop individual ablations within a defined time span;
adjust one or more composite ablation blending parameters; or
enter or select an exact time of activation, deactivation, or both for each of the plural applicators.

9. The system of any one of the preceding claims, wherein the one or more processors are further configured by the instructions to receive an indication when each of the plural applicators is activated.

10. The system of any one of the preceding claims, wherein the one or more processors are further configured by the instructions to determine which of the plural applicators are sequentially activated and simultaneously activated based on a blending parameter.

11. The system of claim 10, wherein the one or more processors are further configured by the instructions to determine at least one of the plural ablation zones is under sequential activation using a single applicator based on the blending parameter having a first value, and determine at least one of another one of the plural ablation zones is under simultaneous activation using a group of the plural applicators based on the blending parameter having a second value different than the first value.

12. The system of any one of the preceding claims, wherein the one or more processors are further configured by the instructions to guide ablation therapy treatment to a tumor corresponding to a target region based on the sequentially activated applicators and simultaneously activated applicators.

13. The system of any one of the preceding claims, wherein the system comprises a medical device.

14. A method of operating a system of any of the preceding claims, wherein the method comprises:

- receiving an ablation plan comprising plural ablation zones corresponding to plural applicators, the plural ablation zones comprising one or more single ablation zones and one or more composite ablation zones; and
- providing on a display device a representation of the ablation plan and a visual distinction between sequentially activated applicators and simultaneously activated applicators, the plural applicators comprising the sequentially activated and simultaneously activated applicators.

15. A non-transitory, computer readable storage medium comprising instructions that when executed by the one or more processors, causes the one or more processors to perform the method of claim 14.

**Patentansprüche**

1. System (12), umfassend:

eine Anzeigevorrichtung (24);

einen Speicher (20), der Anweisungen (32) umfasst; und
einen oder mehrere Prozessoren (16), die durch den Anweisungen konfiguriert sind zum:

Empfangen eines Ablationsplans, der mehrere Ablationszonen umfasst, die mehreren Applikatoren entsprechen, wobei die mehreren Ablationszonen eine oder mehrere einzelne Ablationszonen und eine oder mehrere zusammengesetzte Ablationszonen umfassen; und

Bereitstellen auf der Anzeigevorrichtung einer Darstellung des Ablationsplans (58) und einer visuellen Unterscheidung zwischen sequenziell aktivierten Applikatoren und gleichzeitig aktivierten Applikatoren, wobei die mehreren Applikatoren sowohl die sequenziell aktivierten als auch die gleichzeitig aktivierten Applikatoren umfassen.

2. System nach dem vorstehenden Anspruch, wobei der eine oder die mehreren Prozessoren durch die Anweisungen weiter so konfiguriert sind, dass sie die visuelle Unterscheidung basierend auf Farbcodierung bereitstellen.

3. System nach einem der vorstehenden Ansprüche, wobei die sequenziell aktivierten Applikatoren Einzelablationen bereitstellen und die gleichzeitig aktivierten Applikatoren zusammengesetzte Ablationen bereitstellen, wobei die zusammengesetzten Ablationen auf Überblendung impliziter Funktionen basieren.

4. System nach einem der vorstehenden Ansprüche, wobei der eine oder die mehreren Prozessoren durch die Anweisungen weiter so konfiguriert sind, dass sie eine Anpassung an einer oder mehreren der mehreren Ablationszonen basierend auf einer Änderung des Zustands des Ablationsplans vornehmen, wobei die Anpassung eines oder eine Kombination aus Neuberechnungen der einen oder mehreren zusammengesetzten Ablationszonen oder der einen oder mehreren einzelnen Ablationszonen umfasst.

5. System nach einem der vorstehenden Ansprüche, wobei der eine oder die mehreren Prozessoren durch die Anweisungen weiter so konfiguriert sind, dass sie vom Benutzer angeforderte Anpassungen des Ablationsplans, die nicht durchführbar sind, unterbinden.

6. System nach Anspruch 5, wobei die vom Benutzer angeforderten Anpassungen gleichzeitig aktivierte Applikatoren für Pull-Back-Ablationen, eine Ablation basierend auf einem nicht in Betrieb genommenen Applikator oder gleichzeitig aktivierte Applikatoren, deren Anzahl größer ist als eine Anzahl verfügbarer Kanäle umfassen.

7. System nach einem der vorstehenden Ansprüche, wobei der eine oder die mehreren Prozessoren durch die Anweisungen weiter so konfiguriert sind, dass sie eine grafische Benutzeroberfläche (56A, 56B) bereitstellen, die es einem Benutzer ermöglicht, Ablationen zu steuern, die den mehreren Ablationszonen des Ablationsplans entsprechen.

8. System nach Anspruch 7, wobei es die grafische Benutzeroberfläche einem Benutzer ermöglicht, eines oder eine Kombination von Folgendem durchzuführen:

Markieren einer geplanten Gruppe von Ablationen als abgeschlossen oder abgetragen;
Starten oder Stoppen einer geplanten Gruppe von Ablationen;
Markieren einer Auswahl mehrerer Ablationen als abgeschlossen oder abgetragen;
Starten und Stoppen einer Auswahl mehrerer Ablationen;
Starten und Stoppen einzelner Ablationen innerhalb eines definierten Zeitraums;
Anpassen von einem oder mehreren Parametern für Überblendung zusammengesetzter Ablationen; oder
Eingeben oder Auswählen eines genauen Zeitpunkts für Aktivierung, Deaktivierung oder beides für jeden der mehreren Applikatoren.

9. System nach einem der vorstehenden Ansprüche, wobei der eine oder die mehreren Prozessoren durch die Anweisungen weiter so konfiguriert sind, dass sie einen Hinweis erhalten, wenn jeder der mehreren Applikatoren aktiviert wird.

10. System nach einem der vorstehenden Ansprüche, wobei der eine oder die mehreren Prozessoren weiter durch die Anweisungen so konfiguriert sind, dass sie basierend auf einem Überblendungsparameter bestimmen, welche der mehreren Applikatoren sequenziell und welche gleichzeitig aktiviert werden.

11. System nach Anspruch 10, wobei der eine oder die mehreren Prozessoren weiter durch die Anweisungen so konfiguriert sind, dass sie basierend auf dem Überblendungsparameter, der einen ersten Wert aufweist, bestimmen, dass mindestens eine der mehreren Ablationszonen unter Verwendung eines einzelnen Applikators unter sequenzieller Aktivierung ist, und basierend auf dem Überblendungsparameter, der einen zweiten, vom ersten Wert unterschiedlichen Wert aufweist, bestimmen, dass mindestens eine andere der mehreren Ablationszonen unter Verwendung einer Gruppe der mehreren Applikatoren unter gleichzeitiger Aktivierung ist.

12. System nach einem der vorstehenden Ansprüche, wobei der eine oder die mehreren Prozessoren weiter durch die Anweisungen so konfiguriert sind, dass sie die Ablationstherapiebehandlung eines Tumors, der einer Zielregion entspricht, basierend auf den sequenziell aktivierten Applikatoren und den gleichzeitig aktivierten Applikatoren steuern.

13. System nach einem der vorstehenden Ansprüche, wobei das System eine medizinische Vorrichtung umfasst.

14. Verfahren zum Betreiben eines Systems nach einem der vorstehenden Ansprüche, wobei das Verfahren Folgendes umfasst:

Empfangen eines Ablationsplans, der mehrere Ablationszonen umfasst, die mehreren Applikatoren entsprechen, wobei die mehreren Ablationszonen eine oder mehrere einzelne Ablationszonen und eine oder mehrere zusammengesetzte Ablationszonen umfassen; und
Bereitstellen auf einer Anzeigevorrichtung einer Darstellung des Ablationsplans und einer visuellen Unterscheidung zwischen sequenziell aktivierten Applikatoren und gleichzeitig aktivierten Applikatoren, wobei die mehreren Applikatoren sowohl die sequenziell aktivierten als auch die gleichzeitig aktivierten Applikatoren umfassen.

15. Nichtflüchtiges, computerlesbares Speichermedium, das Anweisungen umfasst, die, wenn sie von dem einem oder den mehreren Prozessoren ausgeführt werden, bewirken, dass der eine oder die mehreren Prozessoren das Verfahren nach Anspruch 14 durchführen.


**Revendications**

1. Système (12) comprenant :

un dispositif d'affichage (24) ;
une mémoire (20) comprenant des instructions (32) ; et
un ou plusieurs processeurs (16) configurés par les instructions pour :

recevoir un plan d'ablation comprenant plusieurs zones d'ablation correspondant à plusieurs applicateurs, les plusieurs zones d'ablation comprenant une ou plusieurs zones d'ablation simples et une ou plusieurs zones d'ablation composites ; et
afficher sur le dispositif d'affichage une représentation du plan d'ablation (58) et une distinction visuelle entre des applicateurs activés séquentiellement et des applicateurs activés simultanément, les plusieurs applicateurs comprenant les applicateurs activés séquentiellement et les applicateurs activés simultanément.

2. Système selon la revendication précédente, dans lequel les un ou plusieurs processeurs sont en outre configurés par les instructions pour fournir la distinction visuelle sur la base d'un codage couleur.

3. Système selon l'une quelconque des revendications précédentes, dans lequel les applicateurs activés séquentiellement fournissent des ablations simples et les applicateurs activés simultanément fournissent des ablations composites, dans lequel les ablations composites sont basées sur le mélange de fonctions implicites.

4. Système selon l'une quelconque des revendications précédentes, dans lequel les un ou plusieurs processeurs sont en outre configurés par les instructions pour effectuer un ajustement à une ou plusieurs des plusieurs zones d'ablation sur la base d'un changement d'état du plan d'ablation, dans lequel l'ajustement comprend un ou une combinaison de recalculs des une ou plusieurs des zones d'ablation composites ou des une ou plusieurs des zones d'ablation simples.

5. Système selon l'une quelconque des revendications précédentes, dans lequel les un ou plusieurs processeurs sont en outre configurés par les instructions pour interdire des ajustements demandés par l'utilisateur au plan d'ablation qui sont irréalisables.

6. Système selon la revendication 5, dans lequel les ajustements demandés par l'utilisateur incluent des applicateurs activés simultanément pour des ablations par retrait, une ablation basée sur un applicateur non mis en service ou des applicateurs activés simultanément qui sont plus nombreux qu'un nombre de canaux disponibles.

7. Système selon l'une quelconque des revendications précédentes, dans lequel les un ou plusieurs processeurs sont en outre configurés par les instructions pour fournir une interface utilisateur graphique (56A, 56B) qui permet à un utilisateur de commander des ablations correspondant aux plusieurs zones d'ablation du plan d'ablation.

8. Système selon la revendication 7, dans lequel l'interface utilisateur graphique permet à un utilisateur d'effectuer une ou une quelconque combinaisons des actions suivantes :

marquer un groupe d'ablations planifié comme étant terminées ou ablatées ;
démarrer et arrêter un groupe d'ablations planifié ;
marquer une sélection d'ablations multiples comme terminées ou ablatées ;
démarrer et arrêter une sélection d'ablations multiples ;
démarrer et arrêter des ablations individuelles dans un laps de temps défini ;
ajuster un ou plusieurs paramètres de mélange d'ablations composites ; ou
saisir ou sélectionner une heure précise d'activation, de désactivation ou les deux pour chacun des plusieurs applicateurs.

9. Système selon l'une quelconque des revendications précédentes, dans lequel les un ou plusieurs processeurs sont en outre configurés par les instructions pour recevoir une indication lorsque chacun des plusieurs applicateurs est activé.

10. Système selon l'une quelconque des revendications précédentes, dans lequel les un ou plusieurs processeurs sont en outre configurés par les instructions pour déterminer lesquels des plusieurs applicateurs sont activés séquentiellement et simultanément sur la base d'un paramètre de mélange.

11. Système selon la revendication 10, dans lequel les un ou plusieurs processeurs sont en outre configurés par les instructions pour déterminer qu'au moins une des plusieurs zones d'ablation est activée séquentiellement à l'aide d'un seul applicateur sur la base du paramètre de mélange présentant une première valeur et pour déterminer qu'au moins une autre des plusieurs zones d'ablation est activée simultanément à l'aide d'un groupe des plusieurs applicateurs sur la base du paramètre de mélange présentant une seconde valeur différente de la première valeur.

12. Système selon l'une quelconque des revendications précédentes, dans lequel les un ou plusieurs processeurs sont en outre configurés par les instructions pour guider le traitement de thérapie par ablation vers une tumeur correspondant à une région cible sur la base des applicateurs activés séquentiellement et des applicateurs activés simultanément.

13. Système selon l'une quelconque des revendications précédentes, dans lequel le système comprend un dispositif médical.

14. Procédé de fonctionnement d'un système selon l'une quelconque des revendications précédentes, dans lequel le procédé comprend :

- la réception d'un plan d'ablation comprenant plusieurs zones d'ablation correspondant à plusieurs applicateurs, les plusieurs zones d'ablation comprenant une ou plusieurs zones d'ablation simples et une ou plusieurs zones d'ablation composites ; et
- la fourniture sur un dispositif d'affichage d'une représentation du plan d'ablation et d'une distinction visuelle entre des applicateurs activés séquentiellement et des applicateurs activés simultanément, les plusieurs applicateurs comprenant les applicateurs activés séquentiellement et les applicateurs activés simultanément.

15. Support de stockage non transitoire lisible par ordinateur comprenant des instructions qui, lorsqu'elles sont exécutées par les un ou plusieurs processeurs, amènent les un ou plusieurs processeurs à mettre en œuvre le procédé selon la revendication 14.

FIG. 1A

10A →

FIG. 1B

10B →

FIG. 1C

10C →

FIG. 2

**FIG. 3**

**FIG. 4A**

**FIG. 4B**

FIG. 5A

FIG. 5B

FIG. 5C

44

START

46 COMPUTE GROUPS OF ENTRY TRAJECTORIES

48 GENERATE ABLATION CONFIGURATIONS USING THE COMPUTED GROUPS

50 SELECT A PREFERRED SET OF COMPOSITE ABLATION ZONES

52 GUIDE PLACEMENT OF APPLICATORS BASED ON SELECTED COMPOSITE ABLATION ZONES

54 PERFORM THERMAL ABLATION THERAPY

END

FIG. 6

**FIG. 7A**

EP 4 687 721 B1

**FIG. 7B**

**REFERENCES CITED IN THE DESCRIPTION**

**Patent documents cited in the description**

- US 2021007805 A
- WO 2021032449 A